# FASCICULE DE BREVET EUROPEEN

(11) **EP 0 674 887 B1**
(45) Date de publication et mention de la délivrance du brevet: **27.10.1999**
(21) Numéro de dépôt: 95400643.3
(22) Date de dépôt: 23.03.1995
(51) Int. Cl.: A61F 2/38

(54) **Perfectionnement aux prothèses de genou**
Verbesserung an Knieprothesen
Improvement in knee prosthesis

(30) Priorité: 29.03.1994 FR 9403645
(43) Date de publication de la demande: 04.10.1995
(73) Titulaire: Bouvet, Jean-Claude, F-91590 Mondeville (FR)
(72) Inventeur: Bouvet, Jean-Claude, F-91590 Mondeville (FR); Husson, Jean-Louis, F-35000 Rennes (FR); Garcia, Alain, F-79000 Niort (FR); Hy, Xavier, F-49000 Angers (FR); Le Huec, Jean-Charles, F-33700 Merignac (FR); Landjerit, Bernard, F-60290 Monchy st Eloi (FR); Leroy, Paul, F-11100 Narbonne (FR); Letournel, Emile, F-92000 Neuilly-sur-Seine (FR)
(74) Mandataire: Flavenot, Bernard

(56) Documents cités:
- EP-A- 0 519 873
- FR-A- 2 691 356

## Description

La présente invention concerne un perfectionnement aux prothèses de genou qui permettent de restaurer l'articulation entre le fémur, le tibia et la rotule, par exemple aux prothèses de genou décrites dans le FR-A-2 691 356.

Dans le document référencé ci-dessus, qui doit être compris comme faisant partie intégrante de la présente description, il est décrit une prothèse comportant une pièce fémorale apte à être fixée sur le fémur et épousant sensiblement la forme d'un "U", le fond et l'un des côtés dudit "U" définissant deux surfaces condyliennes séparées par un espace vide pour le passage de ligaments, l'autre côté dudit "U" définissant deux surfaces de trochlée, un plateau tibial apte à être fixé sur le tibia et étant constitué par deux plaques et par des moyens pour monter en rotation les deux plaques l'une sur l'autre autour d'un axe sensiblement parallèle à l'axe du tibia, une première plaque étant apte à être fixée sur le tibia et la seconde plaque étant tournée vers le fémur, et des moyens pour coupler les deux surfaces condyliennes avec le plateau tibial constitués par deux patins disposés entre respectivement les deux surfaces condyliennes et la seconde plaque dudit plateau tibial, les deux patins étant montés coulissants en translation suivant, respectivement, deux axes rectilignes parallèles et comportant chacun une surface de glissement en rotation pour la surface condylienne qui lui est associée, chaque surface condylienne étant constituée, pour une partie, par une surface cylindrique de révolution et, pour son autre partie, par une surface torique de révolution, les surfaces cylindrique de révolution et torique de chaque surface condylienne étant juxtaposées l'une à l'autre en formant une continuité sans point anguleux, la surface de glissement en rotation de chaque patin étant congruente d'une partie des deux surfaces cylindrique et torique de la surface condylienne associée au patin.

La présente invention a pour but de réaliser un perfectionnement aux prothèses de genou décrites dans le brevet FR-A-2 691 356.

Plus précisément, la présente invention a pour objet une prothèse de genou comportant:
- une pièce fémorale apte à être fixée sur le fémur, la pièce fémorale épousant sensiblement la forme d'un "U", le fond et l'un des côtés dudit "U" définissant deux surfaces condyliennes ces deux surfaces condyliennes étant séparées par un espace vide pour le passage de ligaments, l'autre côté dudit "U" définissant deux surfaces de trochlée,
- un plateau tibial apte à être fixé sur le tibia, ledit plateau tibial étant constitué par deux plaques et par des moyens pour monter en rotation les deux dites plaques l'une sur l'autre autour d'un axe sensiblement parallèle à l'axe du tibia, une première plaque étant apte à être fixée sur ledit tibia et la seconde plaque étant tournée vers le fémur, et
- des moyens pour coupler les deux dites surfaces condyliennes avec ledit plateau tibial constitués par deux patins disposés entre respectivement les deux surfaces condyliennes et la seconde plaque dudit plateau tibial, et par des moyens pour monter les deux dits patins coulissants en translation par rapport à la seconde plaque suivant, respectivement, deux axes rectilignes et parallèles, chaque patin comportant une surface de glissement en rotation pour la surface condylienne qui lui est associée, chaque surface condylienne étant constituée, pour une partie, par une surface cylindrique de révolution et, pour son autre partie, par une surface torique de révolution, les surfaces cylindrique de révolution et torique de chaque surface condylienne étant juxtaposées l'une à l'autre en formant une continuité sans point anguleux et la surface de glissement en rotation de chaque patin étant congruente d'une partie des deux dites surfaces cylindrique et torique de la surface condylienne associée audit patin,
   caractérisée par le fait que chaque patin est constitué par une enveloppe et un noyau enchâssé dans ladite enveloppe, la surface de glissement en rotation de chaque patin étant réalisée sur ledit noyau, et que lesdits moyens pour monter chaque patin coulissant en translation par rapport à ladite seconde plaque sont constitués par une lumière rectiligne réalisée dans l'un des deux éléments "seconde plaque" et "enveloppe" suivant l'un des dits axes et par un tenon solidaire de l'autre des deux dits éléments.

D'autres caractéristiques et avantages de la présente invention apparaîtront au cours de la description suivante donnée en regard des dessins annexés à titre illustratif mais nullement limitatif, dans lesquels:
La figure 1 représente, en vue de face, un premier mode de réalisation d'une prothèse selon l'invention, dans une application pour le genou droit,
La figure 2 représente une vue en coupe, référencée II-II sur la figure 1, d'une prothèse selon l'invention dans le premier mode de réalisation,
Les figures 3 et 4 représentent une coupe, référencée III-III sur la figure 1, d'un second mode de réalisation d'une prothèse selon l'invention dans deux positions du tibia par rapport au fémur, respectivement en extension et en flexion,
Les figures 5 et 6 représentent un mode de réalisation du plateau tibial entrant dans la constitution de la prothèse, respectivement dans une vue de dessus et en coupe référencée VI-VI sur la figure 5, et
La figure 7 représente un détail de réalisation d ' un perfectionnement apporté à la prothèse selon les figures 3 et 4.

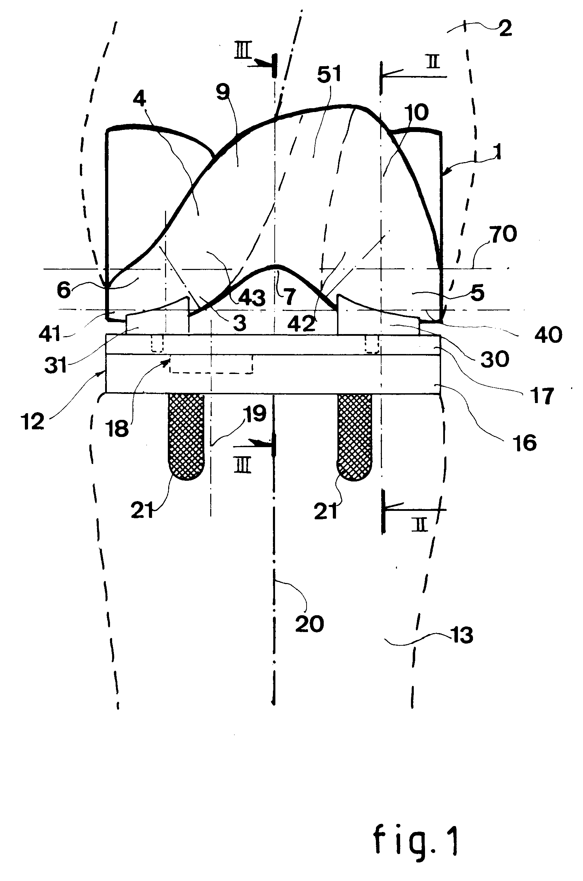

Bien que les figures 1 à 4 représentent deux modes de réalisation d'une prothèse de genou, les mêmes références y désignent les mêmes éléments, quelle que soit la forme de représentation de ces éléments et quelle que soit la figure sur laquelle ils sont représentés.

La prothèse de genou selon l'invention comporte une pièce fémorale 1 apte à être fixée sur le fémur 2. Cette pièce fémorale épouse sensiblement la forme d'un "U" dont le fond 3 et l'un des côtés 4 définissent deux surfaces condyliennes 5, 6 séparées par un espace vide 7 pour le passage des ligaments, l'autre côté 8 du "U" définissant deux surfaces de trochlée 9, 10.

La prothèse de genou comporte aussi un plateau tibial 12 apte à être fixé sur le tibia 13 et des moyens 14 pour coupler les deux surfaces condyliennes 5, 6 avec le plateau tibial 12.

Le plateau tibial 12 est constitué par deux plaques 16, 17 et par des moyens 18 pour monter en rotation ces deux plaques l'une sur l'autre autour d'un axe 19 sensiblement parallèle à l'axe 20 du tibia lors de la mise en place de la prothèse. Une première plaque 16 est apte à être fixée sur le tibia par tous moyens, par exemple une ou plusieurs pattes 21, la seconde plaque 17 étant tournée vers le fémur 2.

Quant aux moyens 14 pour coupler les deux surfaces condyliennes 5, 6 avec le plateau tibial 12, plus particulièrement la seconde plaque 17, ils sont constitués par deux patins 30, 31 disposés entre, respectivement, les deux surfaces condyliennes 5, 6 et la seconde plaque 17 du plateau tibial 12, chaque patin comportant une surface de glissement en rotation 34, 35 pour la surface condylienne 5, 6 qui lui est associée, et par des moyens pour monter ces deux patins 30, 31 coulissants en translation par rapport à la seconde plaque 17 suivant, respectivement, deux axes rectilignes et parallèles 32, 33.

Dans un mode de réalisation avantageux, chaque surface condylienne 5, 6 est constituée, pour une partie, par une surface cylindrique de révolution, les deux surfaces cylindriques de révolution ayant le même axe de révolution, et, pour son autre partie, par une surface torique de révolution ou analogue, les surfaces cylindrique de révolution et torique de chaque surface condylienne étant juxtaposées l'une à l'autre en formant une continuité sans point anguleux au niveau de leur jonction. Avantageusement, les deux surfaces toriques condyliennes sont disposées en regard l'une de l'autre en bordant de chaque côté l'espace vide ligamenteux 7.

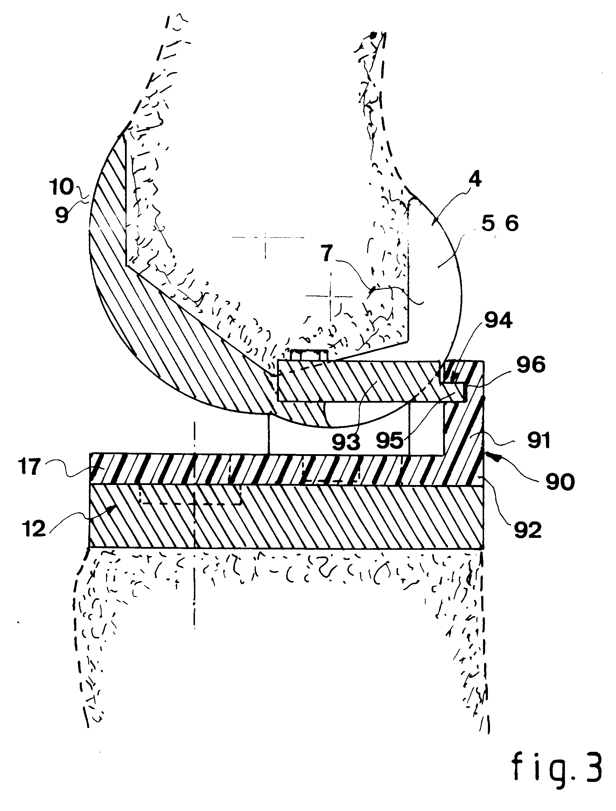

La surface de glissement en rotation 34, 35 de chaque patin est congruente d'une partie des deux surfaces cylindrique et torique de la surface condylienne associée au patin et comporte donc, elle aussi, des surfaces cylindrique de révolution 46, 47 et torique 48, 49, figure 3.

En fait, les surfaces cylindriques de révolution des patins et les surfaces condyliennes permettent d'obtenir une rotation parfaite du plateau tibial 12 et de la pièce fémorale 1 l'un par rapport à l'autre, tandis que les surfaces toriques permettent de maintenir transversalement ces deux pièces et éviter qu'elles ne se translatent l'une par rapport à l'autre suivant une direction parallèle à l'axe 70 des deux surfaces cylindriques condyliennes et des patins.

Par ailleurs, les deux surfaces de trochlée 9, 10 sont séparées par une surface rotulienne en creux 51, et une partie de chacune d'elles comporte au moins une portion de surface cylindrique de révolution 52, 53 sensiblement en continuité d'une surface cylindrique de révolution condylienne 40, 41, les surfaces cylindriques de révolution de trochlée et condylienne se coupant en formant sensiblement un dièdre, la surface cylindrique de trochlée 52, 53 formant, au niveau de l'arête du dièdre, des moyens de butée 50 entre les surfaces condyliennes 5, 6 et de trochlée 9, 10.

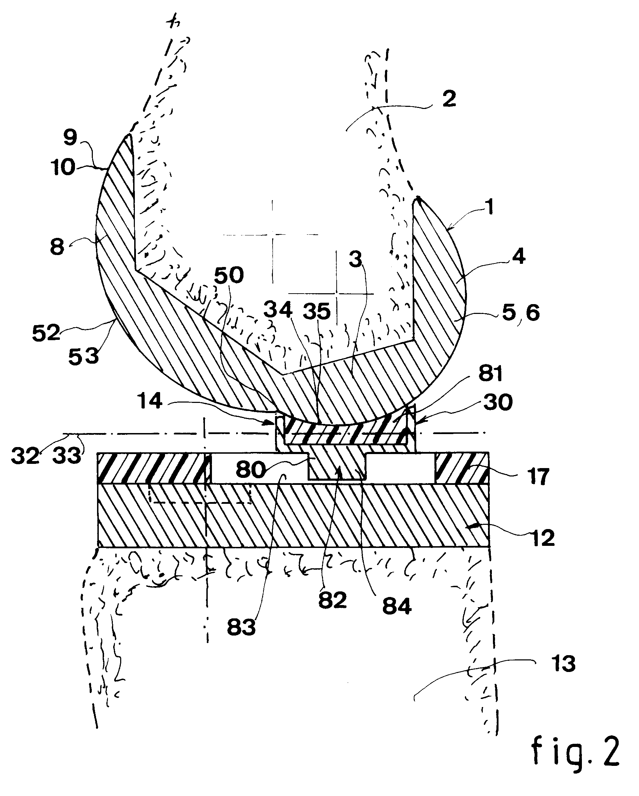

Dans une premier mode de réalisation avantageux plus particulièrement représenté sur la figure 2, chaque patin 30, 31 est constitué par une enveloppe 80 et un noyau 81 enchâssé dans l'enveloppe, la surface de glissement en rotation 34, 35 de chaque patin étant réalisée sur le noyau 81. quant aux moyens 82 pour monter chaque patin coulissant en translation par rapport à la seconde plaque 17, ils sont constitués par une lumière rectiligne 83 réalisée dans l'un des deux éléments "seconde plaque 17" et "enveloppe 80" suivant l'un des axes 32, 33 et par un tenon 84 solidaire de l'autre des deux éléments. Dans le mode de réalisation illustré, la lumière 83 est réalisée dans la seconde plaque 17 tandis que le tenon 84 est solidaire de l'enveloppe 80.

Dans ces conditions, l'enveloppe 80 glisse sur la seconde plaque 17 en s'appuyant sur les bords latéraux de la lumière 83, le tenon 84 ne servant que de guidage dans la translation du patin.

Pour que les patins ne se dissocient pas de la seconde plaque 17, la prothèse comporte en outre des moyens pour enclipser en translation chacun des tenons par rapport à la seconde plaque 17. Ces moyens sont connus en eux-même et peuvent être constitués, pour chaque tenon, par un clip solidaire du tenon et coopérant avec une rainure réalisée dans la seconde plaque.

Selon un mode de réalisation préférentiel en accord avec la structure décrite ci-avant, la pièce fémorale 1, les noyaux 81, les enveloppes 80, la seconde plaque 17 et la première plaque 16 sont respectivement réalisés dans des premier, deuxième, troisième, quatrième et cinquième matériaux, et le coefficient de dureté du premier matériau est supérieur à celui du deuxième matériau, le coefficient de dureté du troisième matériau est supérieur à celui du quatrième matériau et le coefficient de dureté du quatrième matériau est inférieur à celui du cinquième matériau.

Ces dernières caractéristiques facilitent le déplacement, les unes par rapport aux autres, des différentes pièces mobiles entrant dans la constitution de la prothèse.

Par exemple, les premier, troisième et cinquième matériaux sont des matériaux métalliques comme du titane ou analogue, tandis que les deuxième et quatrième matériaux sont des matériaux plastiques ou analogues.

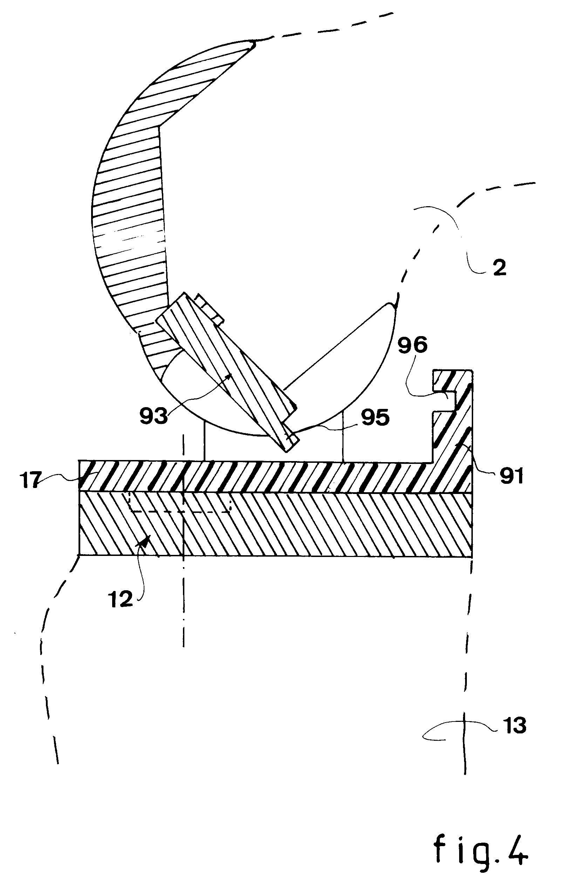

La prothèse de genou selon l'invention dans le mode de réalisation décrit ci-dessus en regard des figures 1 et 2 répond parfaitement aux conditions imposées pour restaurer l'articulation du genou dans le cas où les ligaments du genou ont tous pu être conservés et stabilisent correctement la position du tibia par rapport au fémur. Elle est cependant parfaitement avantageuse, également, pour restaurer l'articulation d'un genou dont les deux ligaments croisés antérieur et postérieur sont très abîmés ou même sectionnés, dans le cas où elle est réalisée selon le mode représenté sur les figures 3 et 4.

Selon le mode de réalisation décrit ci-après en regard des figures 3 et 4, la prothèse comporte en outre des moyens de butée 90 pour déterminer une position limite entre la pièce fémorale 1 et la plateau tibial 12, plus particulièrement la seconde plaque 17, lorsque le fémur 2 et le tibia 13 viennent en position d'extension, figure 3.

De préférence, ces moyens de butée 90 comportent une première partie de butée 91 solidaire d'une partie arrière 92 de la seconde plaque 17 par rapport au tibia 13, et une seconde partie de butée 93 située sur la pièce fémorale 1 dans l'espace vide 7 entre les deux surfaces condyliennes 5, 6.

En outre, dans le but d'obtenir une bonne stabilité entre le fémur et le tibia dans leur position d'extension, semblable à celle existant dans l'articulation naturelle du genou, les première et seconde parties de butée 91, 93 coopèrent entre elles par des moyens d'emboîtement 94 qui sont par exemple du type "mâle-femelle" 95, 96, la partie mâle 95 étant avantageusement réalisée dans la seconde partie de butée 93 et la partie femelle 96 réalisée dans la première partie de butée 91.

Quand la prothèse décrite ci-dessus est implantée sur un patient, elle fonctionne, d'une façon générale, comme décrit dans le FR-A-2 691 356. Dans le but de simplifier la présente description, il ne sera donc décrit ici que la partie de fonctionnement relative au mouvement d'extension du tibia par rapport au fémur dans le cas où, par exemple pour les raisons mentionnées ci-avant concernant l'état des ligaments, la prothèse implantée est réalisée selon les figures 3 et 4.

Dans ce cas, lorsque le tibia arrive en fin de rotation vers l'avant, c'est-à-dire en position d'extension (figure 3) par rapport au fémur, les moyens de butée 90 empêchent le tibia d'avancer par rapport au fémur et les moyens d'emboîtement "mâle-femelle" 94 confortent le maintien de la pièce fémorale 1 par rapport au plateau tibial 12, évitant ainsi tout risque de déboîtement du genou.

Ces moyens de butée 90 et les moyens d'emboîtement 94 confèrent donc à la prothèse une parfaite sécurité de fonctionnement, même dans le cas où les ligaments croisés antérieur et postérieur ont été sectionnés.

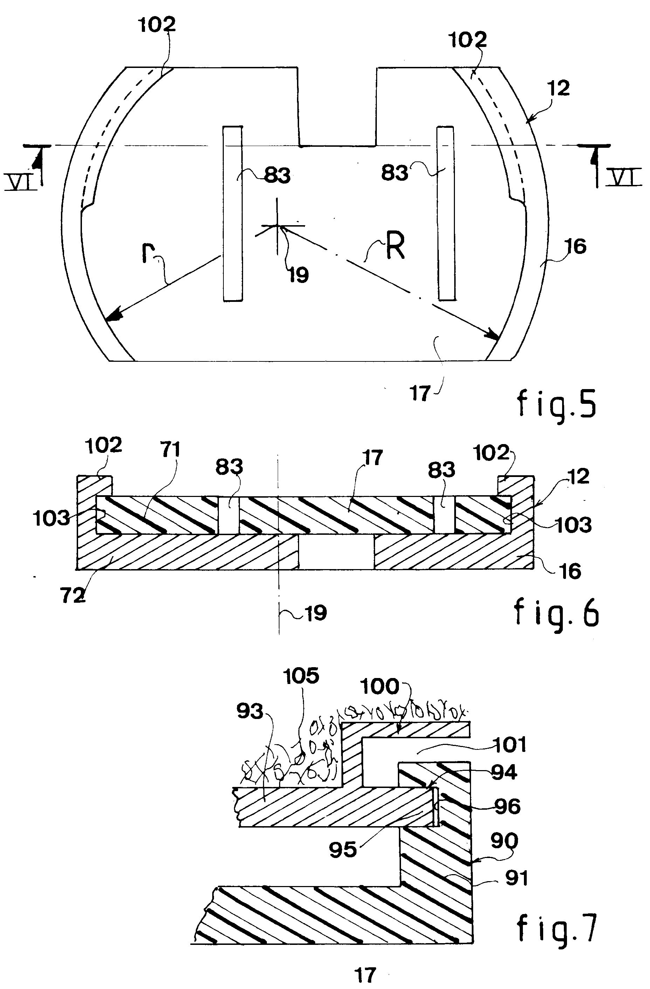

La figure 7 représente un perfectionnement à la réalisation de la prothèse selon les figures 3 et 4. Dans ce perfectionnement, la prothèse comporte en outre un capot de protection 100 des moyens d'emboîtement mâles 95 pour définir un espace libre 101 entre ces moyens d'emboîtement mâles et le fémur 2. En effet, lorsque la pièce fémorale est placée sur le fémur, il est souvent utilisé du ciment de scellement 105, en plus de pattes de fixation. Le capot de protection 100 évite ainsi que du ciment 105 ne vienne obturer l'espace autour de cette partie mâle 95 et n'empêche les deux parties mâle 95 et femelle 96 de coopérer par emboîtement comme décrit ci-avant.

Les figures 5 et 6, quant à elles, représentent un mode de réalisation perfectionné, par rapport au mode de réalisation illustré sur les figures 1 à 4, du plateau tibial 12 entrant dans la constitution de la prothèse, respectivement dans une vue de dessus et en coupe.

Selon ce perfectionnement, l'axe de rotation 19 des deux première 16 et seconde 17 plaques l'une sur l'autre et l'axe 20 du tibia 13 sont parallèles et distants l'un de l'autre d'une valeur sensiblement comprise entre le sixième et le dixième de la largeur de la seconde plaque 17 prise suivant une direction parallèle à l'axe de révolution 70 des surfaces cylindriques de révolution condyliennes 40, 41 définies ci-avant, et les moyens 18 pour monter en rotation les deux plaques 16, 17 l'une sur l'autre autour de l'axe 19 sont constitués par une partie mâle 71 solidaire de l'une 17 des deux plaques et comportant deux portions cylindriques de révolution de même axe réalisées de façon que le rayon "R" de l'une ait une valeur sensiblement comprise entre le double et les trois demis de la valeur du rayon "r" de l'autre, et par une partie femelle 72 réalisée dans l'autre plaque 16, complémentaire de la partie mâle.

Dans l'exemple illustré, la partie mâle 71 est réalisée dans la seconde plaque 17, et c'est même cette seconde plaque qui la constitue dans sa totalité, les deux lumières 83 définies ci-avant pour le guidage des tenons 84 étant visibles sur ces figures 5 et 6.

Il est de plus avantageux que la plaque, en l'occurrence la première plaque 16, dans laquelle est réalisée la partie femelle 72 comporte au moins une bordure 102 recouvrant partiellement cette partie femelle 72 pour constituer une rainure d'enclipsage 103 de la partie mâle 71 complémentaire de cette partie femelle. Selon le mode de réalisation illustré, la prothèse comporte deux bordures 102 disposées sur la partie arrière de la première plaque 16 par rapport au tibia, pour que le praticien chargé de l'opération puisse, tout d'abord implanter la première plaque 16 sur le tibia 13, puis glisser la seconde plaque 17, par l'avant du genou et sous les bordures 102, le genou étant en position fléchie, jusqu'à ce que cette seconde plaque soit enclipsée dans la rainure 103. La seconde plaque est ainsi parfaitement maintenue dans la première, même lors d'une flexion importante du tibia par rapport au fémur, ce qui évite le risque de luxation du genou. Cette réalisation confère à la prothèse selon l'invention encore un avantage important par rapport à toutes les prothèses de genou connues de l'art antérieur.

## Revendications

1. Prothèse de genou comportant:
- une pièce fémorale (1) apte à être fixée sur le fémur (2), la pièce fémorale épousant sensiblement la forme d'un "U", le fond (3) et l'un (4) des côtés dudit "U" définissant deux surfaces condyliennes (5, 6), ces deux surfaces condyliennes étant séparées par un espace vide (7) pour le passage de ligaments, l'autre côté dudit "U" définissant deux surfaces de trochlée (9, 10),
- un plateau tibial (12) apte à être fixé sur le tibia (13), ledit plateau tibial étant constitué par deux plaques (16, 17) et par des moyens pour monter en rotation les deux dites plaques l'une sur l'autre autour d'un axe (19) sensiblement parallèle à l'axe (20) du tibia, une première plaque (16) étant apte à être fixée sur ledit tibia et la seconde plaque (17) étant tournée vers le fémur (2), et
- des moyens pour coupler les deux dites surfaces condyliennes (5, 6) avec ledit plateau tibial (12) constitués par deux patins (30, 31) disposés entre respectivement les deux surfaces condyliennes (5, 6) et la seconde plaque (17) dudit plateau tibial (12), et par des moyens pour monter les deux dits patins (30, 31) coulissants en translation par rapport à la seconde plaque (17) suivant, respectivement, deux axes rectilignes et parallèles (32, 33), chaque patin comportant une surface de glissement en rotation (34, 35) pour la surface condylienne qui lui est associée, chaque surface condylienne (5, 6) étant constituée, pour une partie, par une surface cylindrique de révolution (40,41) et, pour son autre partie, par une surface torique de révolution (42, 43), les surfaces cylindrique de révolution et torique de chaque surface condylienne étant juxtaposées l'une à l'autre en formant une continuité sans point anguleux et la surface de glissement en rotation de chaque patin étant congruente d'une partie des deux dites surfaces cylindrique et torique de la surface condylienne associée audit patin,
CARACTERISEE PAR LE FAIT QUE chaque patin (30, 31) est constitué par une enveloppe (80) et un noyau (81) enchâssé dans ladite enveloppe, la surface de glissement en rotation (34, 35) de chaque patin étant réalisée sur ledit noyau (81), et que lesdits moyens (82) pour monter chaque patin coulissant en translation par rapport à ladite seconde plaque (17) sont constitués par une lumière rectiligne (83) réalisée dans l'un des deux éléments "seconde plaque" et "enveloppe" suivant l'un des dits axes (32, 33) et par un tenon (84) solidaire de l'autre des deux dits éléments.

2. Prothèse selon la revendication 1, caractérisée par le fait que la pièce fémorale (1), les noyaux (81), les enveloppes (80), la seconde plaque (17) et première plaque (16) sont respectivement réalisés dans des premier, deuxième, troisième, quatrième et cinquième matériaux, le coefficient de dureté du premier matériau étant supérieur à celui du deuxième matériau, le coefficient de dureté du troisième matériau étant supérieur à celui du quatrième matériau et le coefficient de dureté du quatrième matériau étant inférieur à celui du cinquième matériau.

3. Prothèse selon la revendication 2, caractérisée par le fait que les premier, troisième et cinquième matériaux sont des matériaux métalliques et que les deuxième et quatrième matériaux sont des matériaux plastiques.

4. Prothèse selon l'une des revendications 1 à 3, caractérisée par le fait qu'elle comporte en outre des moyens de butée (90) pour déterminer une position limite entre ladite pièce fémorale (1) et ladite seconde plaque (17) lorsque le fémur (2) et le tibia (13) viennent en position d'extension.

5. Prothèse selon la revendication 4, caractérisée par le fait que lesdits moyens de butée (90) comportent une première partie de butée (91) solidaire d'une partie arrière (92) de ladite seconde plaque (17) par rapport au tibia (13), et une seconde partie de butée (93) située sur la pièce fémorale (1) dans l'espace vide (7) entre les deux surfaces condyliennes (5, 6).

6. Prothèse selon la revendication 5, caractérisée par le fait que les première et seconde parties de butée (91, 93) coopèrent entre elles par des moyens d'emboîtement (94).

7. Prothèse selon la revendication 6, caractérisée par le fait que les moyens d'emboîtement (94) sont du type "mâle-femelle" (95, 96).

8. Prothèse selon l'une des revendications 6 et 7, caractérisée par le fait que lesdits moyens d'emboîtement mâles (95) sont réalisés sur la partie de butée (93) qui est solidaire de la pièce fémorale (1), et qu'elle comporte en outre un capot de protection (100) de ces moyens d'emboîtement mâles (95) définissant un espace libre (101) entre ces moyens d'emboîtement mâles et le fémur.

9. Prothèse selon l'une des revendications 1 à 8, caractérisée par le fait que l'axe de rotation (19) des deux dites plaques (16, 17) l'une sur l'autre et l'axe (20) du tibia (13) sont parallèles et distants l'un de l'autre d'une valeur sensiblement comprise entre le sixième et le dixième de la largeur de la seconde plaque (17) prise suivant une direction parallèle à l'axe de révolution (70) des surfaces cylindriques de révolution condyliennes (40, 41), et que les moyens (18) pour monter en rotation les deux dites plaques (16, 17) l'une sur l'autre autour dudit axe (19) sensiblement parallèle à l'axe (20) du tibia (13) sont constitués par une partie mâle (71) solidaire de l'une (17) des deux plaques, ladite partie mâle comportant deux portions cylindriques de révolution de même axe réalisées de façon que le rayon (R) de l'une ait une valeur sensiblement comprise entre le double et les trois demis de la valeur du rayon (r) de l'autre, et par une partie femelle (72) réalisée dans l'autre plaque (16), complémentaire de la partie mâle.

10. Prothèse selon la revendication 9, caractérisée par le fait que la plaque (16) dans laquelle est réalisée la partie femelle (72) entrant dans la constitution des moyens (18) pour monter en rotation les deux plaques l'une sur l'autre comporte au moins une bordure (102) recouvrant partiellement ladite partie femelle (72) pour constituer une rainure d'enclipsage (103) de la partie mâle (71) complémentaire de cette partie femelle (72).

## Claims

1. A knee prosthesis comprising:
• a femur piece (1) suitable for being fixed to the femur (2), the femur piece being substantially U-shaped, the bottom (3) and one of the sides (4) of the U-shape defining two condyle surfaces (5, 6), the two condyle surfaces being separated by an empty space (7) for passing ligaments, the other side of said U-shape defining two trochlea surfaces (9, 10);
• a tibia plateau (12) suitable for being fixed on the tibia (13), said tibia plateau being constituted by two plates (16, 17) and by means for mounting the said two plates to rotate relative to each other about an axis (19) that is substantially parallel to the axis (20) of the tibia, a first plate (16) being suitable for being fixed on said tibia and the second plate (17) facing towards the femur (2); and
• means for coupling said two condyle surfaces (5, 6) with said tibia plateau (12), said means being constituted by two skids (30, 31) disposed between respective ones of the two condyle surfaces (5, 6) and the second plate (17) of said tibia plateau (12), and by means for mounting said two skids (30, 31) to slide in translation relative to the second plate (17) along two respective rectilinear and parallel axes (32, 33), each skid having a rotary sliding surface (34, 35) for the associated condyle surface, each condyle surface (5, 6) being constituted in part by a circularly cylindrical surface (40, 41) and in part by a circularly toroidal surface (42, 43), the circularly cylindrical and toroidal surfaces of each condyle surface being juxtaposed to each other to form continuity without any angular point, and the rotary sliding surface of each skid being congruent with a portion of said two cylindrical and toroidal surfaces of the condyle surface associated with said skid;
the prosthesis being characterized by the facts that each skid (30, 31) is constituted by a covering (80) and a core (81) engaged in said covering, the rotary sliding surface (34, 35) of each skid being made on said core (81), and that said means (82) for mounting each skid to slide in translation relative to said second plate (17) are constituted by a rectilinear slot (83) extending along one of said axes (32, 33) and formed in one of the two elements comprising the second plate and the covering, and by a tenon (84) secured to the other one of said two elements.

2. A prosthesis according to claim 1, characterized by the fact that the femur piece (1), the cores (81), the coverings (80), the second plate (17), and the first plate (16) are made respectively out of first, second, third, fourth, and fifth materials, the coefficient of hardness of the first material being greater than that of the second material, the coefficient of hardness of the third material being greater than that of the fourth material, and the coefficient of hardness of the fourth material being less than that of the fifth material.

3. A prosthesis according to claim 2, characterized by the fact that the first, third, and fifth materials are metal materials and the second and fourth materials are plastics materials.

4. A prosthesis according to any one of claims 1 to 3, characterized by the fact that it further includes abutment means (90) for defining a limit position between said femur piece (1) and said second plate (17) when the femur (2) and the tibia (13) come into the extension position.

5. A prosthesis according to claim 4, characterized by the fact that said abutment means (90) comprise a first abutment part (91) secured to a rear portion (92) of said second plate (17) relative to the tibia (13), and a second abutment part (93) situated on the femur piece (1) in the empty space (7) between the two condyle surfaces (5, 6).

6. A prosthesis according to claim 5, characterized by the fact that the first and second abutment parts (91, 93) co-operate with each other via engagement means (94).

7. A prosthesis according to claim 6, characterized by the fact that the engagement means (94) are of the "male-female" type (95, 96).

8. A prosthesis according to claim 6 or 7, characterized by the facts that said male engagement means (95) are formed on the abutment part (93) which is situated on the femur piece (1), and that it also includes a protective cap (100) for protecting said male engagement means (95), defining an empty space (101) between said male engagement means and the femur.

9. A prosthesis according to any one of claims 1 to 8, characterized by the fact that the axis (19) of relative rotation between said two plates (16, 17) and the axis (20) of the tibia (13) are parallel and spaced apart from each other by a distance lying substantially in the range one-sixth to one-tenth of the width of the second plate (17) as measured in a direction parallel to the axis of revolution (70) of the circularly cylindrical condyle surfaces (40, 41), and that the means (18) for mounting said two plates (16, 17) to rotate relative to each other about said axis (19) that is substantially parallel to the axis (20) of the tibia (13) are constituted by a male part (71) integral with one of the two plates (17), said male part having two circularly cylindrical portions about the same axis made in such a manner that the radius (R) of one of them lies substantially in the range two times to three-and-a-half times the radius (r) of the other one of them, and by a female part (72) formed in the other plate (16) and complementary to the male part.

10. A prosthesis according to claim 9, characterized by the fact that the plate (16) having formed therein the female part (72) involved in the structure of the means (18) for mounting the two plates to rotate relative to each other includes at least one margin (102) partially overlying said female part (72) to constitute a snap-fastening groove (103) for the male part (71) complementary to said female part (72).

## Patentansprüche

1. Knieprothese, mit:
- einem Oberschenkelteil (1), das am Oberschenkelknochen (2) befestigt werden kann und im wesentlichen die Form eines "U" hat, wobei der Boden (3) und eine (4) der Seiten des "U" zwei Gelenkkopfoberflächen (5, 6) definieren, die durch einen leeren Zwischenraum (7) für den Durchgang von Ligamenten voneinander getrennt sind, wobei die andere Seite des "U" zwei Trochlea-Oberflächen (9, 10) definiert,
- einer Schienbeinplatte (12), die am Schienbein (13) befestigt werden kann, wobei die Schienbeinplatte aus zwei Platten (16, 17) sowie aus Mitteln gebildet ist, mit denen die beiden Platten um eine zur Achse (20) des Schienbeins im wesentlichen parallele Achse (19) drehbar angebracht sind, wobei eine erste Platte (16) am Schienbein befestigt werden kann und die zweite Platte (17) dem Oberschenkelknochen (2) zugewandt ist, und
- Mitteln zum Koppeln der beiden Gelenkkopfoberflächen (5, 6) mit der Schienbeinplatte (12), die aus zwei Gleitschuhen (30, 31), die jeweils zwischen einer entsprechenden der beiden Gelenkkopfoberflächen (5, 6) und der zweiten Platte (17) der Schienbeinplatte (12) angeordnet sind, sowie aus Mitteln zum Anbringen der beiden Gleitschuhe (30, 31) in der Weise, daß sie in Translationsrichtung in bezug auf die zweite Platte (17) jeweils längs einer entsprechenden von zwei geradlinigen und parallelen Achsen (32, 33) gleiten, gebildet sind, wobei jeder Gleitschuh eine Rotationsgleitoberfläche (34, 35) für die ihr zugeordnete Gelenkkopfoberfläche aufweist, wobei jede Gelenkkopfoberfläche (5, 6) einerseits aus einer rotationssymmetrischen zylindrischen Oberfläche (40, 41) und andererseits aus einer rotationssymetrischen torischen Oberfläche (42, 43) gebildet ist, wobei die rotationssymmetrischen zylindrischen und torischen Oberflächen jeder Gelenkkopfoberfläche nebeneinanderliegen und einen stetigen Verlauf ohne Kante bilden und die Rotationsgleitoberfläche jedes Gleitschuhes zu einem Teil der beiden zylindrischen und torischen Oberflächen der Gelenkkopfoberfläche, der dem Gleitschuh zugeordnet ist, kongruent ist,
DADURCH GEKENNZEICHNET, DASS jeder Gleitschuh (30, 31) aus einer Hülle (80) und einem in die Hülle eingelassenen Kern (81) gebildet ist, wobei die Rotationsgleitoberfläche (34, 35) jedes Gleitschuhes am Kern (81) ausgebildet ist, und daß die Mittel (82), mit denen jeder Gleitschuh in der Weise angebracht ist, daß er in Translationsrichtung in bezug auf die zweite Platte (17) gleitet, aus einem geradlinigen Langloch (83), das in einem der beiden Elemente "zweite Platte" und "Hülle" längs einer der Achsen (32, 33) ausgebildet ist, und aus einem Zapfen (84), der mit dem anderen dieser beiden Elemente fest verbunden ist, gebildet sind.

2. Prothese nach Anspruch 1, dadurch gekennzeichnet, daß das Oberschenkelteil (1), die Kerne (81), die Hüllen (80), die zweite Platte (17) und die erste Platte (16) aus einem ersten, einem zweiten, einem dritten, einem vierten bzw. einem fünften Material hergestellt sind, wobei der Härtekoeffizient des ersten Materials größer als derjenige des zweiten Materials ist, der Härtekoeffizient des dritten Materials größer als derjenige des vierten Materials ist und der Härtekoeffizient des vierten Materials kleiner als derjenige des fünften Materials ist.

3. Prothese nach Anspruch 2, dadurch gekennzeichnet, daß die ersten, dritten und fünften Materialien metallische Materialien sind und die zweiten und vierten Materialien Kunststoffe sind.

4. Prothese nach einem der Ansprüche 1 bis 3, dadurch gekennzeichnet, daß sie außerdem Anschlagmittel (90) enthält, um die Grenzposition zwischen dem Oberschenkelteil (1) und der zweiten Platte (17) festzulegen, wenn der Oberschenkelknochen (2) und das Schienbein (13) in die Streckposition gelangen.

5. Prothese nach Anspruch 4, dadurch gekennzeichnet, daß die Anschlagmittel (90) ein erstes Anschlagteil (91), das mit einem hinteren Teil (92) der zweiten Platte (17) in bezug auf das Schienbein (13) verbunden ist, und ein zweites Anschlagteil (93), das sich am Oberschenkelteil (1) im leeren Raum (7) zwischen den beiden Gelenkkopfoberflächen (5, 6) befindet, enthalten.

6. Prothese nach Anspruch 5, dadurch gekennzeichnet, daß die ersten und zweiten Anschlagteile (91, 93) über Einfügungsmittel (94) zusammenwirken.

7. Prothese nach Anspruch 6, dadurch gekennzeichnet, daß die Einfügungsmittel (94) vom Typ ''Stecker-Buchse'' (95, 96) sind.

8. Prothese nach einem der Ansprüche 6 und 7, dadurch gekennzeichnet, daß die Stecker-Einfügungsmittel (95) an dem Anschlagteil (93), das mit dem Oberschenkelteil (1) fest verbunden ist, verwirklicht sind und daß sie außerdem eine Schutzhaube (100) für diese Stecker-Einfügungsmittel (95) enthält, die zwischen diesen Stecker-Einfügungsmitteln und dem Oberschenkelknochen einen freien Raum (101) definieren.

9. Prothese nach einem der Ansprüche 1 bis 8, dadurch gekennzeichnet, daß die Drehachse (19), um die sich die beiden Platten (16, 17) drehen, und die Achse (20) des Schienbeins (13) zueinander parallel und voneinander um einen Wert beabstandet sind, der im wesentlichen im Bereich von einem Sechstel und bis zu einem Zehntel der Breite der zweiten Platte (17) in einer zur Symetrieachse (17) der rotationssymmetrischen zylindrischen Gelenkkopf-Oberflächen (40, 41) parallelen Richtung liegt, und daß die Mittel (18), mit denen die beiden Platten (16, 17) um eine zur Achse (20) des Schienbeins (13) im wesentlichen parallele Achse (19) drehbar angebracht sind, aus einem Steckerteil (71), das mit einer (17) der beiden Platten fest verbunden ist und zwei rotationssymmetrische zylindrische Abschnitte mit derselben Achse enthält, die in der Weise verwirklicht sind, daß der Radius (R) des einen Abschnitts einen Wert besitzt, der im wesentlichen im Bereich vom doppelten bis eineinhalbfachen Wert des Radius (r) des anderen Abschnitts liegt, sowie aus einem Buchsenteil (72), das in der anderen Platte (16) verwirklicht ist und zum Steckerteil komplementär ist, gebildet sind.

10. Prothese nach Anspruch 9, dadurch gekennzeichnet, daß die Platte (16), in der der Buchsenteil (72) verwirklicht ist, das in die Bildung der Mittel (18), mit denen die beiden Platten umeinander drehbar angebracht sind, eingeht, wenigstens einen Rand (102) enthält, die das Buchsenteil (72) teilweise abdeckt, um eine Einrastnut (103) für den zu diesem Buchsenteil (72) komplementären Steckerteil (71) zu bilden.
